(19) Europäisches Patentamt
European Patent Office
Office européen des brevets

(11) EP 1 570 902 A1

(12) EUROPÄISCHE PATENTANMELDUNG

(43) Veröffentlichungstag:
**07.09.2005 Patentblatt 2005/36**

(51) Int Cl.[7]: **B01J 19/24**

(21) Anmeldenummer: **05100167.5**

(22) Anmeldetag: **13.01.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **06.03.2004 DE 102004011081**

(71) Anmelder: **Oxeno Olefinchemie GmbH**
**45772 Marl (DE)**

(72) Erfinder:
- **Wiese, Klaus-Diether**
  **45721, Haltern am See (DE)**
- **Büschken, Wilfried**
  **45721, Haltern am See (DE)**

(54) **Verfahren zur Herstellung von tertiären Carbonsäuren**

(57) Verfahren zur katalytischen Durchführung von Mehrphasenreaktionen in einem Rohrreaktor, bei denen zumindest drei Edukte in drei unterschiedlichen Phasen vorliegen, wobei das Verfahren dadurch gekennzeichnet ist, dass das Verfahren in zumindest einem Rohrreaktor durchgeführt wird, wobei der Katalysator in einer kontinuierlichen Phase enthalten ist, zumindest ein Edukt in dieser kontinuierlichen Phase enthalten ist, und zumindest zwei Edukte in der kontinuierlichen Phase dispergiert enthalten sind und der Belastungsfaktor B des Rohrreaktors gleich oder größer 0,2 ist. Insbesondere ist die Mehrphasenreaktion eine Hydrocarboxylierung zumindest eines Olefins zu einer Carbonsäure.

EP 1 570 902 A1

## Beschreibung

Die vorliegende Erfindung betrifft ein Verfahren zur Durchführung einer Mehrphasenreaktion an der zumindest drei Edukte beteiligt sind, insbesondere ein Verfahren zur Herstellung von Carbonsäuren durch Hydrocarboxylierung von Olefinen.

Tert.-Carbonsäuren werden für die Herstellung von Sikkativen, Peroxiden und Schmiermitteln eingesetzt. Ein weiteres Einsatzgebiet von tertiären Carbonsäuren ist die Herstellung von ungesättigten Estern, wie Vinylestern, aus denen Cooligomere oder Copolymere hergestellt werden.

Seit den 60-er-Jahren ist die direkte Carbonylierung von Olefmen mit Kohlenmonoxid zu Carbonsäuren unter Verwendung von starken Säuren als Katalysator (Koch reactions, in New Synthesis with Carbon Monoxide, J. Falbe (Ed.), Springer, Berlin, 1980, p. 372) bekannt. Später wurde entdeckt, dass die Reaktion zusätzlich durch Silber- oder Kupfer-Kationen katalysiert werden kann (Y. Souma, H. Sano, Bull. Chem. Soc. Jpn. 1974, 47, 1717). Bei diesen Reaktionen entstehen unabhängig von der Struktur des Ausgangsolefms vorwiegend tertiäre Carbonsäuren, wenn das Ausgangsolefin eine auseichend große Anzahl von Kohlenstoffatomen aufweist (n in der unten angegebenen Formel größer 3):

$$C_nH_{2n} + CO + H2O \quad \underset{\text{Katalysator}}{} \quad R^1R^2R^3C\text{-}COOH \qquad \text{mit } (R^1 + R^2 + R^3) = C_{n-1}\,H_{2n+1}$$

Technisch werden diese Reaktionen üblicherweise in gerührten Reaktoren bei 40 bis 70 °C und 70 bis 100 bar CO-Druck mit $BF_3/H_2O$ oder $BF_3/H_3PO_4/H_2O$ als Katalysator durchgeführt. Durch die Verwendung von Cu-Ionen aufweisenden Katalysatoren lässt sich die Reaktion schon bei Umgebungstemperatur und Normaldruck durchführen.

Nachteilig bei allen bekannten Verfahren ist, dass wegen des geringen Stoffiibergangs zwischen den Phasen die Raum-Zeit-Ausbeuten gering sind und dass ein Teil der eingesetzten Olefine oligomerisiert und die Oligomerisate teilweise zu höheren Carbonsäuren umgesetzt werden.

Aufgabe der vorliegenden Erfindung war es deshalb ein Verfahren bereitzustellen welches zumindest einen der genannten Nachteile nicht aufweist.

Technisch gesehen handelt es sich bei den oben genannten Prozessen zur Herstellung von Carbonsäuren um Mehrphasenreaktionen. Unter Mehrphasenreaktionen werden Reaktionen verstanden, die unter Beteiligung von zwei oder mehr nicht oder nur teilweise mischbaren fluiden Phasen ablaufen. Dies betrifft z. B. Reaktionen zwischen einer Gas- und einer Flüssigphase (g1), zwischen zwei Flüssigphasen, die nicht mischbar sind oder eine Mischungslücke aufweisen (11) und Reaktionen, an denen sowohl zwei flüssige nicht oder nur teilweise mischbare Phasen sowie eine Gasphase beteiligt sind (g11).

Beispiele für technisch wichtige Gas-Flüssig-Reaktionen (g1) sind neben der Hydroformylierung von flüssigen Olefinen mit einem in organischer Phase gelöstem Katalysator die Reaktion von Acetylen mit Carbonsäuren oder Hydrierungen mit homogen gelösten Katalysatoren oder Oxidationen mit Luft oder Sauerstoff.

Den genannten Reaktionen ist das Problem des Stoffiibergangs gemeinsam, da die Reaktionspartner in unterschiedlichen Phasen vorliegen. Mehrphasenreaktionen sind mit einer Reihe von weiteren Problemen verbunden, die ihre technische Ausführung wesentlich schwieriger machen als bei einfachen homogenen Reaktionen. Im Folgenden sind einige typische Probleme erwähnt:

In allen Fällen müssen die Stoffe möglichst innig miteinander in Kontakt gebracht werden, um das Problem des Stoffiibergangs zu minimieren: Es muss eine möglichst große Stoffübergangsfläche $a_s$ zwischen den Phasen erzeugt werden. Andererseits müssen die Phasen nach erfolgter Reaktion wieder leicht getrennt werden können: Zu starke Vermischung kann hier zu Problemen führen. Bei Anwesenheit zweier flüssiger Phasen kann es zur Emulsionsbildung kommen, bei Gas-Flüssig-Verfahren zum Schäumen. Bei dem hier vorliegenden erwähnten 3-Phasenverfahren können sogar alle Probleme gleichzeitig auftreten.

Neben einer hohen Stoffübergangsftäche $a_s$ sollte in allen Mehrphasenreaktionen ein möglichst hoher Stoffübergangskoeffizient $k_1$ erreicht werden. Insgesamt sollte der sogenannte KLA-Wert, d.h. das Produkt aus $k_1$ und $a_s$ in der Stoffübergangsgleichung

$$j = k_1 * aS * (C* - C)$$

mit

j [Mol/s] der durch die Phasengrenzfläche hindurchtretende Molenstrom der reagierenden Komponente,

$k_1$ [m/s] Stoffübergangskoeffizient,
$a_s$ [$m^2$] Phasengrenzfläche im Reaktor,
$C*$ [$Mol/m^3$] maximale Löslichkeit des Edukts in der zweiten Phase und
$C$ [$Mol/m^3$] tatsächliche Konzentration des Edukts, die wiederum mit der Reaktionsgeschwindigkeit gekoppelt ist,

maximal sein.

Ein weiteres Problem bei Mehrphasenreaktionen ist die Wärmeabfuhr bei exothermen Reaktionen. Gelingt es, die Reaktionsgeschwindigkeit durch Verbesserung des Stoffüberganges zu erhöhen, muss naturgemäß auch mehr Wärme abgeführt werden, was zu unerwünschter Temperaturerhöhung bis hin zum Durchgehen der Reaktion führen kann.

Diese Problematik der Mehrphasenreaktion kann technisch z. B. durch die Verwendung eines Rührkesselreaktors gelöst werden. Die Verwendung eines Rührkessels führt jedoch immer zu einer Rückvermischung, wodurch die effektive Konzentration der Reaktanden herabgesetzt wird, was zur Absenkung der Raumzeitausbeute führt. Dieser Nachteil muss mit der Investition von teurem Reaktionsraum bezahlt werden.

Im Falle der Koch-Reaktion (Umsatz von Olefinen mit CO und Wasser zu Carbonsäuren) im 3-Phasensystem ist die Verfahrensführung besonders schwierig, da die Edukte in drei getrennten Phasen vorliegen. Sowohl Olefin als auch Synthesegas müssen in die wässrige Katalysatorphase transportiert werden, um dort mit dem Katalysator in Kontakt zu kommen. Schließlich muss der Rücktransport aus der wässrigen Phase erfolgen. Da die Transportvorgänge häufig langsamer sind als die eigentliche Reaktion, sind solche Reaktionen durch die Geschwindigkeit des Stoffüberganges bestimmt, man spricht von einer transportgehemmten Reaktion.

Aus den voranstehenden Ausführungen kann entnommen werden, dass ein Bedarf für ein Verfahren zur Durchführung von Mehrphasenreaktionen vorhanden ist, das die vorstehend genannten Nachteile vermeidet und sich zudem auf einfache Weise technisch realisieren lässt.

Aufgabe der vorliegenden Erfindung ist somit auch die Entwicklung eines Verfahrens zur Durchführung von Mehrphasenreaktionen, das sich besonders für die Herstellung von (tertiären) Carbonsäuren durch Hydrocarboxylierung (Koch-Reaktion) von Olefinen eignet.

Technisch sollte das neue Verfahren eine oder möglichst alle Anforderungen an ein Mehrphasenverfahren erfüllen:

- Erzeugung eines hohen und stabilen Stoffübergangs zwischen den beteiligten Phasen
- Einfach ausführbar, möglichst mit üblichen technischen Apparaten
- Einfache und sichere Wärmeabfuhr
- Hohe Betriebssicherheit
- Einfache und sichere Maßstabsübertragung

In Bezug auf die durchzuführende Herstellung von tertiären Carbonsäuren kommen speziell eine oder mehrere der folgenden Anforderungen hinzu:

- Hohe Selektivität, Vermeidung insbesondere hochsiedender Nebenprodukte
- Keine oder nur geringfügige Katalysatorausschleusung
- Hohe Raum-Zeit-Ausbeute, kleine Reaktoren
- Hohe Produktreinheit

Verfahren zur Durchführung von Mehrphasenreaktionen, bei denen zwei Edukte in unterschiedlichen Phasen vorliegen sind für einige Umsetzungen bekannt. So wird in DE 199 25 385 die Umsetzung von Acetylen mit Carbonsäuren zu Vinylestern, in DE 199 25384 und DE 199 57 528 die Hydroformylierung von Olefinen zu Aldehyden, in DE 199 57 522 die Herstellung von ungesättigten Aldehyden durch Aldolkondensation von Aldehyden und in DE 101 06 186 die Herstellung von ungesättigten Ketonen durch Aldolkondensation von Aldehyden mit Ketonen beschrieben.

Allen diesen Verfahren ist gemeinsam, dass die Umsetzung kontinuierlich in einem Rohrreaktor durchgeführt wird, in dem eine kontinuierliche Katalysatorphase vorliegt, worin die Reaktanden dispergiert sind, und dass ein Mindestbelastungsfaktor B eingehalten wird.

Überraschenderweise wurde nun gefunden, dass die Durchführung einer Mehrphasenreaktion in einem Rohrreaktor unter Einhaltung eines Mindestbelastungsfaktors B auch zur Durchführung von Mehrphasenreaktionen geeignet ist, bei denen zumindest drei Edukte in unterschiedlichen Phasen vorliegen. Insbesondere wurde überraschenderweise gefunden, dass Olefine mit Wasser und Kohlenmonoxid im Rohrreaktor bei Einhaltung des Mindestbelastungsfaktors unter hohen Raum-Zeit-Ausbeuten und/oder mit hohen Selektivitäten zu Carbonsäuren umgesetzt werden können. Dies war insbesondere deshalb überraschend, weil bei dieser Reaktion ein Edukt, nämlich das Wasser, homogen in der Katalysatorphase gelöst ist, während das Einsatzolefin in einer flüssigen organischen Phase und das

Kohlenmonoxid in der Gasphase vorliegt.

Gegenstand der vorliegenden Erfindung ist deshalb ein Verfahren zur Durchführung von Mehrphasenreaktionen, bei denen zumindest drei Edukte in drei unterschiedlichen Phasen vorliegen, in einem Rohrreaktor, welches dadurch gekennzeichnet ist, dass das Verfahren in zumindest einem Rohrreaktor durchgeführt wird, wobei der Katalysator in einer kontinuierlichen Phase enthalten ist, zumindest ein Edukt in dieser kontinuierlichen Phase enthalten ist, und zumindest zwei Edukte in der kontinuierlichen Phase dispergiert enthalten sind und der Belastungsfaktor B des Rohrreaktors gleich oder größer 0,2 ist. Insbesondere ist Gegenstand der vorliegenden Erfindung ein Verfahren zur katalytischen Herstellung von Carbonsäuren durch Mehrphasenreaktion von zumindest einem Olefin mit Wasser und Kohlenmonoxid in Gegenwart eines Katalysators in einem Rohrreaktor, der einen Belastungsfaktor B von >= 0,2 aufweist.

Das erfindungsgemäße Verfahren hat den Vorteil, dass die Herstellung von Carbonsäuren mit hohen Raum-Zeit-Ausbeuten gelingt. Durch die hohen Raum-Zeit-Ausbeuten können relativ kleine Reaktoren eingesetzt werden, was zu einer deutlich wirtschaftlicheren Verfahrensweise führt. Ebenfalls vorteilhaft sind die relativ hohe Selektivität, insbesondere die Vermeidung von hochsiedenden Nebenprodukten. Durch die geringe Nebenproduktbildung können Carbonsäuren mit hoher Produktreinheit hergestellt werden.

Das erfindungsgemäße Verfahren zur Durchführung von Mehrphasenreaktionen, bei denen zumindest drei Edukte in drei unterschiedlichen Phasen vorliegen, in einem Rohrreaktor wird im Folgenden beschrieben, ohne dass die Erfindung auf diese Ausführungsarten beschränkt sein soll.

Das erfindungsgemäße katalytische Mehrphasenreaktionsverfahren, bei dem zumindest drei Edukte in drei unterschiedlichen Phasen vorliegen, zeichnet sich dadurch aus, dass das Verfahren in zumindest einem Rohrreaktor durchgeführt wird, wobei der Katalysator in einer kontinuierlichen Phase enthalten ist, zumindest ein Edukt in dieser kontinuierlichen Phase enthalten ist, und zumindest zwei Edukte in der kontinuierlichen Phase dispergiert, vorzugsweise in zwei unterschiedlichen dispergierten Phasen, enthalten sind und der Belastungsfaktor B des Rohrreaktors gleich oder größer 0,2 ist. Insbesondere ist Gegenstand der vorliegenden Erfindung ein Verfahren zur katalytischen Herstellung von Carbonsäuren durch Mehrphasenreaktion von zumindest einem Olefin mit Wasser und Kohlenmonoxid in Gegenwart eines Katalysators in einem Rohrreaktor, wobei der Katalysator in der kontinuierlichen Phase vorliegt, in der das Einsatzolefin und Kohlenmonoxid dispergiert sind, und der Belastungsfaktor des Reaktors gleich oder größer 0,2 ist.

Der im erfmdungsgemäßen Verfahren eingesetzte Rohrreaktor kann Füllkörper oder Einbauten enthalten. Füllkörper im Sinne der vorliegenden Erfindung sind beispielsweise: Raschigringe, Sättel, Pallringe, Telleretten, Maschendrahtringe, Maschendrahtgewebe. Beispiele für Einbauten sind Filterplatten, Strombrecher, Kolonnenböden, Lochbleche oder sonstige Mischvorrichtungen. Als Einbauten im Sinne der vorliegenden Erfindung sind aber auch mehrere enge, parallel geschaltete Rohre denkbar, es resultiert also ein Vielrohrreaktor. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, dass der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [$m^3$/s] aller Komponenten unter Betriebsbedingungen, multipliziert mit g = 9,81 m/$S^2$, d. h. PS = (M/V)$*$g, bedeuten.

Anschaulich wäre PS der statische Druck pro Meter eines Mehrphasengemisches in einem senkrecht stehenden Rohr, wenn alle Phasen mit gleicher Geschwindigkeit strömen würden. PS ist eine reine Rechengröße, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengröße, um die Prozessbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte Lal bis Lgb7 sowie im Standardwerk

Heinz Brauer, Grundlagen der Einphasen- und Mehrphasenströmungen, Verlag Sauerländer, Aarau und Frankfurt am Main, 1971, (nachfolgen Brauer et al.) nachgeschlagen werden.

Der Druckverlust PD ist bei der Einphasenströmung durch ein leeres Rohr durch

$$PD = Cw * \rho/2 * w^2/D$$

mit

| | | |
|---|---|---|
| ρ | [kg/m$^3$] | Dichte des strömenden Mediums unter Betriebsbedingungen, |
| w | [m/s] | Strömungsgeschwindigkeit (Volumenstrom/Querschnittsfläche), |
| D | [m] | Rohrdurchmesser und |
| Cw | [-] | Widerstandsbeiwert des durchströmten Rohres |

gegeben.

Bei einer Strömung durch Füllkörper, Schüttungen oder Einbauten ist die Geschwindigkeit w durch die Effektivgeschwindigkeit (w/ψ) sowie der Rohrdurchmesser D durch den hydraulischen Kanaldurchmesser $d_H$ der Füllkörper oder Einbauten zu ersetzen, sodass gilt:

$$PD = Cw * \rho/2 * (w/\psi)^2 * 1/\ d_H$$

mit

| | | |
|---|---|---|
| $d_H$ | [m] | Hydraulischer Kanaldurchmesser |
| ψ | [-] | Leerrohranteil |
| Cw | [-] | Widerstandsbeiwert des durchströmten Apparates mit Füllung |

Die füllkörperspezifischen Daten $d_H$ und ψ sind häufig Bestandteil der Lieferspezifikationen von Füllkörpern. Für eine Reihe von Füllkörpern sind Daten im oben erwähnten VDI-Wärmeatlas angegeben.

Der Leerrohranteil ψ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F [m$^2$/m$^3$] der Füllkörper oder Einbauten (in der Regel bekannt oder experimentell bestimmbar) nach der einfachen Beziehung

$$d_H = 4\psi/F.$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig n = 1, m = 0 (Ansatz nach S. Ergun, Chem. Engng. Progr. 48, (1948), 89), oder n =1, m = 0,1 (Ansatz nach Brauer et al.) verwendet wird. $K_1$, $K_2$ sind füllkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als

$$Re = w * (\rho/\eta) * D \text{ für Leerrohre bzw.}$$

$Re = (w/\psi) * (\rho/\eta\}) * d_H$ für Rohre mit Einbauten oder Füllkörpern, $\eta$ [Pa*s] bezeichnet jeweils die Viskosität und $\rho$ [kg/m$^3$] die Dichte des strömenden Mediums.

Der Druckverlust bei Zweiphasenströmungen (hier gas-flüssig für Kohlenmonoxid/Katalysatorlösung) steigt überproportional an. Meist wird nach Lockhart-Martinelli (in Brauer et al.) der Druckverlust der Zweiphasenströmung $P_{1g}$ auf den Druckverlust einer der beiden Phasen bezogen, zum Beispiel auf den Druckverlust der reinen strömenden flüssigen Phase $P_1$, und in Beziehung zu dem Verhältnis des Druckverlustes der beiden als allein strömend gedachten Phasen $P_1$ und $P_g$ gesetzt.

Zur Berechnung von Druckverlusten in Zweiphasenströmungen werden häufig dimensionslose Drücke nach $\phi^2 = P_{1g}/P_1$ und $X^2 = P_1/P_g$ eingesetzt. Der weitere Zusammenhang $\phi^2$ = Funktion($X^2$) ist vielfach untersucht. Beispiele finden sich in folgenden Literaturstellen

Y. Sato, T.Hirose, F. Takahashi, M. Toda: "Pressure Loss and Liquid Hold Up in Packed Bed Reactor with Cocurrent Gas-Liquid Down Flow"; J. Chem. Chem. Eng. Of Japan, Vol 6 (Nr. 2), 1973, 147-152;

D. Sweeney: "A Correlation for Pressure Drop in Two-Phase Concurrent Flow in Packed Beds"; AIChE-Journal, Vol.13,7/1967, 663-669;

V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;

R. P. Larkins, R. P. White, D. W. Jeffrey: "Two-Phase Concurrent Flow in Packed Beds"; AIChE-Journal, Vol 7 (Nr. 2), 6/1961, 231-239 oder

N. Midoux, M. Favier, J.- C. Charpentier: " Flow Pattern, Pressure Loss and Liquid Holdup Data in Gas-Liquid Down-Flow Packed Beds with Foaming and Nonfoaming Liquids"; J. Chem. Eng. Of Japan, Vol 9 (Nr. 5), 1976, 350-356.

Häufig wird für die Berechnung der von Midoux vorgeschlagene Zusammenhang, der für viele Gas-Flüssig-Systeme überprüft wurde, benutzt. Bei nichtschäumenden Systemen ist beispielsweise

$$\phi^2 = 1 + 1/X + 1{,}14/X^{0{,}54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al.

Der Druckverlust der Zweiphasenströmung $P_{g1}$ ergibt sich aus dem experimentell bestimmten oder wie oben erläutert abgeschätzten Druckverlust der reinen strömenden Flüssigphase $P_1$ dann mit

$$P_{g1} = \phi^2 * P_1$$

Im Fall der Dreiphasenströmung, wie im speziellen Fall der Herstellung von tertiären Carbonsäuren durch Hydrocarboxylierung von Olefinen gestaltet sich die Berechung des Druckverlustes noch komplexer. Es ist neben der Kohlenstoffmonoxid- und einer flüssigen Katalysatorphase die Gegenwart einer organischen Flüssigphase zu berücksichtigen. Dieser Problematik kann durch die Bestimmung eines weiteren dimensionslosen Druckes $\phi^2_{org} = P_{g11}/P_{1g}$ Rechnung getragen werden, so dass der Druckverlust wie folgt zu bestimmen ist:

$$P_{g11} = \phi^2 * \phi^2_{org} * P_1$$

Allgemein gilt mit der Reaktorlänge L [m]

$$PD = P_{g1}/L \text{ bzw. } PD = P_{g11}/L$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar und sollte größer oder gleich 0,2, bevorzugt größer oder gleich 0,8, besonders bevorzugt größer oder gleich 1 und ganz besonders bevorzugt größer 3 sein.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben. Vom Verfahren her gibt es im Grunde keine Obergrenze, da mit steigender Leerrohrgeschwindigkeit die Vermischung der Phasen ansteigt. Allerdings steigt mit zunehmender Leerrohrgeschwindigkeit auch die Gefahr der Emulsionsbildung und damit erschwerter Phasentrennung außerhalb des Reaktors. Außerdem erhöht sich mit zunehmender Leerrohrgeschwindigkeit (zweckmäßig erzeugt durch einen Kreislaufstrom der Katalysatorlösung) der Energieaufwand. Somit wird aus technischen und wirtschaftlichen Gründen der Belastungsfaktor bevorzugt nicht beliebig erhöht. Vorzugsweise beträgt der Belastungsfaktor deshalb kleiner 100, bevorzugt kleiner 50 und ganz besonders bevorzugt kleiner 30.

Es ist somit ersichtlich, dass neben den Volumenströmen der einzelnen Phasen bzw. den hieraus abgeleiteten Leerrohrgeschwindigkeiten $w = V/(\pi D^2/4)$ die Apparateabmessungen des Reaktors (Länge L, Durchmesser D) sowie insbesondere die Daten der verwendeten Füllkörper (hydraulischer Durchmesser $d_H$, Leerrohranteil $\Psi$) eine Rolle spielen. Über die richtige Wahl dieser Parameter kann das Verfahren unschwer an die unterschiedlichsten Erfordernisse angepasst werden, wichtig ist nur die Einhaltung der Forderung B >= 0,2, vorzugsweise B >= 0,8, bevorzugt B >= 1, besonders bevorzugt >= 3 oder >=10, und ganz besonders bevorzugt >= 20.

Bei einer langsamen Reaktion kann beispielsweise der hydraulischen Durchmesser der Füllkörper klein bzw. ihre spezifische Oberfläche groß gewählt werden, sodass die geforderten Bedingungen für B schon bei kleinen Strömungsgeschwindigkeiten erreicht werden. Auf diese Weise können ausreichende Verweilzeiten über die Länge eines technisch vernünftig dimensionierten Reaktors erreicht werden. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der bzw. den dispersen Phasen $M_2$, welches vorzugsweise größer 1 beträgt. Im Fall der Hydrocarboxylierung ist der Massenstrom der Katalysatorphase $M_1$ vorzugsweise wesentlich größer als der Massenstrom der dispersen Phasen, d h. der organischen Olefinphase $M_{2a}$ und der Kohlenmonoxid-haltigen Gasphase $M_{2b}$. Im erfindungsgemäßen Verfahren beträgt das Massenverhältnis $M_1/M_2$ der kontinuierlichen Phase ($M_1$) zu den dispersen Phasen ($M_2$) vorzugsweise größer 2, besonders bevorzugt gilt $M_1/M_2 > 10$. Strömungsverhältnisse mit $M_1/M_2 > 100$ sind durchaus möglich und häufig sogar vorteilhaft. Die zumindest zwei Edukte, die in der kontinuierlichen Phase dispergiert enthalten sind, können als eine Phase (Gasphase $M_{2a} + M_{2b} = M_{2(g)}$) dispergiert in der kontinuierlichen Phase vorhanden sein. Diese Variante des erfindungsgemäßen Verfahrens liegt immer dann vor, wenn die Edukte unter den Reaktionsbedingungen beide im gasförmigen Zustand vorliegen. Ebenso ist es möglich dass die zumindest zwei Edukte, die in der kontinuierlichen Phase dispergiert enthalten sind, in zwei unterschiedlichen dispersen Phasen $M_{2a}$ und $M_{2b}$, die jeweils in der kontinuierlichen Phase dispergiert sind, in der kontinuierlichen Phase enthalten sind. Diese Variante des erfindungsgemäßen Verfahrens liegt immer dann vor, wenn bei den Reaktionsbedingungen im Reaktor eines der Edukte als Gas und eines der Edukte als flüssige Phase bzw. in einer flüssigen Phase vorliegt.

Unter der Bedingung $M_1/M_2 > 2$ ist die Katalysatorphase die kontinuierliche Phase, während die dispersen Phasen in feine Blasen bzw. in feine Tropfen zerteilt werden. Im erfindungsgemäßen Verfahren ist es möglich, dass mindestens ein Edukt durch die von der kontinuierlichen Phase in den Rohrreaktor eingebrachte Energie dispergiert wird. Dies führt zu einer Verteilung zumindest eines Edukts in Blasen oder Tropfen innerhalb der kontinuierlichen Katalysatorphase.

Sowohl Blasengröße als auch Tropfchengröße lassen sich mit üblichen ingenieurtechnischen Mitteln abschätzen. Es eignen sich hierfür Ansätze mit dimensionslosen Kennzahlen wie

$$d_S/d_H = k * Re_{g1}(_{g11})^m * We_{g1(g11)}^n$$

mit

| | |
|---|---|
| $d_s$ | Durchmesser der Tropfen bzw. Blasen nach Sauter (in Brauer et al.) |
| $d_H$ | hydraulischer Füllkörperdurchmesser, |
| $Re_{g1(g11)}$ | Reynoldszahl der Mehrphasenströmung = $W_{g1(g11)} * (\rho_1/\eta_1) * (d_H/\Psi)$, |
| $We_{g1(g11)}$ | Weberzahl der Mehrphasenströmung = $W_{g1(g11)}2 * (\rho_1/\sigma_{g1}) * (dH/\Psi^2)$, |
| k, m, n | empirische Konstanten (bekannt oder durch Versuche zu ermitteln), |
| w | Leerrohrgeschwindigkeiten [m/s] = $V/(\pi D^2/4)$, |
| V | Volumenstrom unter Betriebsbedingungen [$m^3/s$], |
| ρ | Dichte unter Betriebsbedingungen [$kg/m^3$], |
| η | Viskosität unter Betriebsbedingungen [Pa*s] und |

$\gamma$ Grenzflächenspannung unter Betriebsbedingungen [N/m]

und den Indices 1 (Flüssigphase), g (Gasphase), g1 (Gas/Flüssig-Zweiphasenströmung) und g11 (Gas/Flüssig/Flüssig-Dreiphasenströmung).

Bei strukturierten Packungen wie Sulzer-SMV oder engen Rohren als Einbauten scheint plausibel, dass ein berechneter Blasen- bzw. Tropfendurchmesser $d_s$ größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H <=1, \text{ vorzugsweise } < 0{,}9.$$

Aus dem berechneten Tropfendurchmesser lässt sich schließlich eine Stoffübergangsfläche nach

$$A_S = 6_{\varphi 1}/d_S \ [m^2/m^3]$$

berechnen.

Ganz analog gilt für die Stoffübergangsfläche der Gasbläschen:

$$As = 6\varphi_g/d_S \ [m^2/m^3]$$

Für den Phasenanteil $\varphi_g$ der dispersen gasförmigen Phase (im Falle der Hydrocarboxylierung sind das Kohlenmonoxid-haltige Gas und die organische Phase dispergiert) kann in die obige Gleichung

$$\varphi_g \sim w_9/ \Sigma w_i$$

gesetzt werden. Dabei bedeutet $\Sigma w_i$ die Summe aller drei Phasen.

Ganz entsprechend gilt für den Phasenanteil $\varphi_1$ der flüssigen Phase:

$$\varphi_1 \sim w_1/\Sigma w_i$$

Die Verweilzeit $\tau$ der den Reaktor durchströmenden Phasen lässt sich angenähert nach $\tau \sim L*\psi/w_{g1}$ berechnen. Die Verweilzeit $\tau$ beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise sehr geringer Anteil an Edukten an der Reaktionsmasse, dadurch bedingt wiederum sehr kurze Verweilzeit - bei vielen Mehrphasenreaktionen überraschend hohe Raum-Zeit-Ausbeuten erzielt. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zulässt.

Das erfindungsgemäße Verfahren kann sehr flexibel den unterschiedlichsten Anforderungen angepasst werden. Für spezielle Anforderungen bieten sich folgende Ausführungsformen des erfindungsgemäßen Verfahrens an:

Der Reaktor kann von oben nach unten oder von unten nach oben oder in anderer Richtung betrieben wird.

Erfordert der Einsatzzweck eine sehr lange Durchmischungszone oder sind Ruhezonen z. B. zur Abnahme von Stoffströmen erforderlich, so kann eine kaskadierende Anordnung von Rohrreaktoren mit Einbauten oder Füllkörpern gewählt werden.

Eine Kaskadierung von Rohrreaktoren oder die alternative Anordnung von gepackten und leeren Rohrabschnitten ist möglich und ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, denkbar. Außerdem können in dem erfindungsgemäßen Verfahren einsetzbare Reaktoren mit einer Mehrfacheinspeisung von Gas über die Reaktorlänge versehen werden, wenn der Gasverbrauch so hoch ist, dass ungünstige Phasenverhältnisse von Gas zu Flüssigkeit bei der Zusammenführung der beiden Phasen vor dem Reaktor resultieren.

Die besonderen Bedingungen des erfindungsgemäßen Verfahrens lassen weitere Ausführungsformen des Verfahrens zu. So kann ein hoher notwendiger Kreislauf der Katalysatorphase bzw. der kontinuierlichen Phase zusätzlich zum Betrieb (Antrieb) einer Strahldüse, die als Flüssigkeitsstrahl-Gasverdichter vor dem eigentlichen Rohrreaktor angeordnet wird, ausgenutzt werden. Dies kann zur gründlichen Vorvermischung der beiden Phasen sowie zur Verdichtung der Gasphase, was die Fahrweise bei höheren Vordrücken im Reaktor ermöglicht, eingesetzt werden. Schließlich wird, wenn man umgekehrt statt der Verdichtung des Gases die Saugwirkung ausnutzt, sogar eine Kreislaufführung von Gas unter gleichzeitiger Vorvermischung der Phasen möglich. Die mit der den Katalysator enthaltende kontinuierliche Phase in den Rohrreaktor eingebrachte Energie kann so zur Dispergierung der Eduktphasen bzw. mindestens eines Edukts eingesetzt werden.

Auch die Wärmeabfuhr bei stark exothermen Reaktionen ist beim erfindungsgemäßen Verfahren unkritisch. Der hohe Durchsatz des Katalysatorkreislaufs wirkt als Wärmeträger, so dass selbst bei adiabatischer Fahrweise des Reaktors nur geringe Temperatardifferenzen auftreten und eine homogene Temperaturverteilung im Reaktor ohne Temperaturspitzen resultiert. Die erzeugte Wärme kann bequem durch einen beliebig im äußeren Katalysatorkreislauf angeordneten, konventionellen Wärmetauscher abgeführt oder zur Energiegewinnung ausgenutzt werden. Zur besseren Wärmeabfuhr kann es unter Umständen günstig sein, den Katalysatorkreislauf noch höher zu fahren (also bei einem höheren B-Wert) als nach den Versuchsergebnissen notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig, Werte von 1 - 2 Gew.-% oder sogar darunter sind möglich.
- Der Katalysator wird geschont, die Desaktivierung ist sehr gering und eine kontinuierliche Ausschleusung kann damit entfallen.

In einer besonderen Ausführungsform des erfindungsgemäßen Verfahrens wird in der Mehrphasenreaktion zumindest ein Olefin zu einer Carbonsäure hydrocarboxyliert. Bei dieser Ausführungsform des erfmdungsgemäßen Verfahrens umfassen die eingesetzten Edukte zumindest Wasser, Kohlenmonoxid und zumindest ein Olefin, wobei das Wasser als Edukt in der kontinuierlichen Phase, und das Kohlenmonoxid und das zumindest eine Olefin dispergiert in der kontinuierlichen Phase vorliegen. Kohlenmonoxid und zumindest ein Olefin können als eine gasförmige Phase dispergiert in der kontinuierlichen Phase vorliegen. Ebenso ist es möglich, dass Kohlenmonoxid und zumindest eine Olefin in zwei unterschiedlichen dispergierten Phasen, also als gasförmige Phase und flüssige Phase vorliegen.

Für die besonders bevorzugte Ausführungsform des erfindungsgemäßen Verfahrens, bei dem durch Hydrocarboxylierung von Olefinen Carbonsäuren, vorzugsweise tertiäre Carbonsäuren hergestellt werden, weist die Katalysatorphase vorzugsweise ein komplexes Gemisch aus $BF_3$, HF, $H_2O$ und zumindest einem Metallion, vorzugsweise Cu-Ionen auf. Weist die Katalysatorphase ein komplexes Gemisch auf, welches als Metall nur Kupfer enthält, so kann dieses komplexe Gemisch (diese komplexe Verbindung) vorzugsweise die nachfolgend angegebene Zusammensetzung aufweisen.

$$CU_aH_{(1-a)}[BF_{(3+b)}(OH)_{(1-b)]}\ [H_2O]_n$$

mit

a = 0,05 bis 0,15
b = 0 bis 0,5 und
n = 0,5 bis 1

Das Katalysatorgemisch kann aus handelsüblichen Chemikalien wie Boroxid, Bortrifluorid, Tetrafluorborsäure, Bortrifluorid-Dihydrat, Fluorwasserstoff, Kupfer oder Kupferverbindungen und gegebenenfalls Wasser durch Zusammenmischen in entsprechenden stöchiometrischen Verhältnissen hergestellt werden.

Darüber hinaus können ähnliche komplexe Flüssigkeiten, bei denen Kupfer teilweise oder ganz durch ein oder mehrere Übergangsmetall(e), insbesondere Silber, ersetzt werden, als Katalysatorflüssigkeit verwendet werden. Der prozentuale Wasseranteil A der Katalysatorphase berechnet sich aus der angegebenen Katalysatorformel, wie folgt:

$$A = 18 * 100 * [(1-a)+n]/\text{Gesamtmolmasse}$$

Als Edukte zur Hydrocarboxylierung können eine oder mehrere olefinische Verbindungen (Olefine) eingesetzt werden. Vorzugsweise wird ein Olefin oder eine Mischung aus mehreren Olefinen mit 4 bis 25 C-Atomen, bevorzugt mit 4 bis 16 C-Atomen eingesetzt. Die Lage der Doppelbindung kann end- oder mittelständig sein. Die Gemische können aus Olefinen gleicher, ähnlicher (± 2) oder deutlich unterschiedlicher (> ±2) Anzahl an Kohlenstoffatomen bestehen. Von den Olefinen, die als Edukte vorhanden sein können, seien beispielsweise genannt: 1- oder 2-Penten, 2-Methylbuten, 1-, 2- oder 3-Hexen, 1-Hepten, lineare Heptene mit innenständiger Doppelbindung, Gemische linearer Heptene, 2- oder 3-Methyl-1-hexen, 1-Octen, lineare Octene mit innenständiger Doppelbindung, Gemische linearer Octene, 2, 3-Methylhepten, 1-Nonen, lineare Nonene mit innenständiger Doppelbindung, Gemische linearer Nonene, 2-, 3- oder 4-Methyl-1-octen, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemisch linearer Hexadecene. Geeignete Edukte sind weiterhin u. a. das bei der Dimerisierung von Propen anfallende isomere Hexen-Gemisch (Dipropen), das bei der Dimerisierung von Butenen anfallende isomere Octen-Gemisch (Dibuten), das bei der Trimerisierung von Propen anfallende isomere Nonen-Gemisch (Tripropen), das bei der Tetramerisierung von Propen oder der Trimerisierung von Butenen anfallende isomere isomere Dodecen-Gemisch (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind isomere Octen-, Nonen-, Dodecen- oder Hexadecen-Gemische, insbesondere solche, die durch Oligomerisation von niedrigen Olefinen, wie n-Butenen oder Propen erhalten wurden. Besonders bevorzugt werden als Olefine $C_8$-Olefine, insbesondere isomere Gemische von $C_8$-Olefinen eingesetzt. Andere gut geeignete Edukte sind Gemische, die im Wesentlichen aus den isomeren $C_5$-Olefinen bestehen.

Darüber hinaus sind Olefme mit einer Methylverzweigung an einem der zweiten C-Atome (von beiden Kettenenden aus gezählt) und einem Isoindex von 1 sowie Gemische, die große Anteile solcher Olefme enthalten, begehrte Einsatzstoffe, da aus ihnen bei der Hydrocarboxylierung tertiäre Carbonsäuren mit zwei Alkylgruppen in 2-Stellung entstehen.

Die eingesetzten Olefme können (unter Normalbedingungen) gasförmig, flüssig oder fest sein. Das erfindungsgemäße Verfahren ist bei Einsatz eines gasförmigen Olefins zunächst eine Zweiphasenreaktion, wobei sich während der Reaktion eine flüssige Produktphase bildet. In allen anderen Fällen liegt bereits zu Beginn der Reaktion ein Dreiphasensystem vor. Feste Olefine werden als Lösungen eingesetzt. Als Lösungsmittel werden inerte, in der Katalysatorphase kaum oder gar nicht lösliche Flüssigkeiten verwendet. Besonders bevorzugt sind Lösungsmittel, die einen deutlich anderen Siedepunkt als die herzustellenden Produkte haben, da hierdurch die destillative Abtrennung und Rückführung erleichtert wird. Auch flüssige Olefine können bevorzugt als Lösungen eingesetzt werden. Die Konzentration des eingesetzten Olefins bzw. die Summe aller eingesetzten Olefine in der Lösung kann dabei von 5 Massen-% bis über 99 Massen-% variieren.

Das zweite Edukt bei dem erfindungsgemäßen Verfahren ist Kohlenmonoxid. Es können reines Kohlenmonoxid oder Kohlenmonoxid-haltige Gasgemische, deren andere Gasbestandteile unter Reaktionsbedingungen inert sind, eingesetzt werden. Geeignete Gasgemische sind beispielsweise Synthesegas oder andere Gemische aus Kohlenmonoxid und Wasserstoff. Weitere inerte Gase in Kohlenmonoxid-Gemischen können beispielsweise Stickstoff, Methan, Ethan oder Propan sein. Der Kohlenmonoxid-Gehalt in diesen Mischungen ist im Allgemeinen keine kritische Größe. Es ist allerdings nicht zweckmäßig, Gase mit einem Kohlenmonoxid-Gehalt unter 10 Gew. % einzusetzen, da dann die Raum-Zeit-Ausbeute zurückgehen und Produkt und Edukt mit dem Abgas verloren gehen könnte(n). Der Einsatz von Synthesegas bietet häufig einen Kostenvorteil, weshalb der Einsatz von Synthesegas besonders bevorzugt ist. Zur Erzielung besonders hoher Selektivitäten kann es vorteilhaft sein, reines Kohlenmonoxid oder Gas-Gemische, die 85 bis 99 Massen-%, bevorzugt 95 bis 99 Massen-% an Kohlenmonoxid aufweisen, einzusetzen.

Das erfindungsgemäße Verfahren wird vorzugsweise so durchgeführt, dass das molare Verhältnis von Kohlenmonoxid zu Olefin (bzw. Summe aller Olefine) am Eingang des Reaktors größer 1 beträgt. Mit zunehmenden Verhältnis von Kohlenmonoxid zu Olefin werden Nebenreaktionen, wie beispielsweise die Oligomerisierung des Einsatzolefin oder der Einsatzolefme, zurückgedrängt. Vorzugsweise wird ein molares Verhältnis zwischen 2 zu 1 bis 5 zu 1 angewendet.

Als drittes Edukt wird Wasser eingesetzt. Dieses ist in der Katalysatorphase in dem eingesetzten Gemisch enthalten.

Die spezifische Verweilzeit, die Massenverhältnisse der Phasen und der Belastungsfaktor sind in weiten Bereichen wählbar. Die einstellbaren Parameter sind dabei abhängig von der Anzahl der Rohrreaktoren sowie das Vorhandensein und die Dimensionierung der eventuell vorhandenen Einbauten abhängig.

Bei der Hydrocarboxylierung von Olefmen, wie z. B. bei der Hydrocarboxylierung von $C_8$-Olefinen kann ein

Massenverhältnis von Katalysatorphase zu Olefinphase (Olefin und gegebenenfalls vorhandenem Lösemittel) am Reaktoreingang im Bereich von 10/1 bis 1000/1, vorzugsweise im Bereich 100/1 bis 700/1, ganz besonders bevorzugt im Bereich von 150/1 bis 500/1 eingestellt werden.

Das Massenverhältnis von Katalysatorphase und Kohlenmonoxid-haltigen Einsatzgas (Einsatzgas und gegebenenfalls vorhandenem Inertgas) kann bei der Hydrocarboxylierung, z. B. der Hydrocarboxylierung von $C_8$-Olefinen beispielsweise von 50 zu 1 bis 1000 zu 1, vorzugsweise von 80 zu 1 bis 700 zu 1 betragen.

Die Reaktanden (Edukte) können vorgewärmt, d. h. mit einer Temperatur im Bereich der Reaktionstemperatur, oder kalt zugeführt werden. Aufgrund des hohen Phasenverhältnisses von Katalysatorphase zu dispergierten Eduktphasen, kann die Vorwärmung auch durch die Prozesswärme erfolgen.

Das erfindungsgemäße Verfahren zur Hydrocarboxylierung von Olefinen erfolgt vorzugsweise in einem Temperaturbereich von 10 °C bis 150 °C, bevorzugt im Bereich von 20 °C bis 60 °C. Der Gesamtdruck, bei dem das erfindungsgemäße Verfahren bevorzugt durchgeführt wird, beträgt von 0,1 bis und 30 MPa, vorzugsweise von 0,1 bis 5 MPa, ganz besonders bevorzugt von 0,5 bis 2 MPa.

Der Rohrreaktor kann im Gleichstrom von oben nach unten oder umgekehrt durchströmt werden. Aus Sicherheitsgründen wird der Beschickung von oben der Vorzug gegeben. Der Rohrreaktor wird vorzugsweise so betrieben, dass eine oder mehrere der im Rohrreaktor vorhandenen Phasen ganz oder teilweise zurückgeführt werden.

Das den Reaktor verlassende Gemisch kann z. B. in einem Gas-Flüssig-Trennbehälter entgast werden. Die Gas-Flüssigkeits-Trennung kann beim gleichen Druck, der auch am Reaktorausgang herrscht, erfolgen. Dies ist besonders dann von Vorteil, wenn zumindest ein Teil des abgetrennten Gases (Entspannungsgases) in den Reaktor zurückgeführt werden soll. Ansonsten kann auch bei tieferem Druck (bis hinunter auf 0,1 MPa) entspannt werden.

Die Rückführung des Abgases in den Reaktor ist im Allgemeinen nur sinnvoll, wenn es zum größten Teil aus Kohlenmonoxid besteht. Dies ist beispielsweise dann der Fall, wenn reines Kohlenmonoxid als Eduktgas eingesetzt wird. Wird beispielsweise Synthesegas als Eduktgas verwendet, besteht das Abgas hauptsächlich aus Wasserstoff. Dieses kann beispielsweise als Heizgas genutzt werden oder zu reinem Wasserstoff aufgearbeitet werden. Das Abgas wird, wenn es nicht in den Rohrreaktor zurückgeführt wird, vor der Zuführung zu einer Aufarbeitung oder Entsorgung vorzugsweise gekühlt und anfallendes Kondensat in den Prozess zurückgeführt oder der Aufarbeitung des gesamten Reaktorprodukts oder der flüssigen Bestandteile des Reaktorprodukts zugeführt. Optional können leichtflüchtige Carbonsäuren mit Wasser aus dem Abgasstrom ausgewaschen werden. Die dabei anfallende Lösung kann beispielsweise als Waschlösung für das Rohprodukt verwendet werden.

Wird das abgetrennte Gas ganz oder teilweise zurückgeführt, so kann dies auf bekannte Weise, z. B. durch eine Mischdüse, die im Katalysatorkreislaufstrom vor dem Reaktor angebracht ist, oder durch einen Kreisgasverdichter, erreicht werden.

Das entgaste Flüssigkeitsgemisch wird vorzugsweise in einem Flüssig-Flüssig-Trennbehälter in die Katalysatorphase und eine Produktphase getrennt. Diese Trennung kann in Absitzbehältern verschiedener Bauart oder Zentrifugen erfolgen. Aus Kostengründen werden Absitzbehälter bevorzugt. Optional kann Gasabtrennung und Trennung der beiden flüssigen Phasen in der gleichen Apparatur durchgeführt werden.

Die abgetrennte Katalysatorlösung wird ganz oder teilweise, vorzugsweise ganz in den Rohrreaktor zurückgeführt. Dabei wird die Katalysatorlösung um das als Edukt bei der Reaktion verbrauchte Wasser ergänzt. Das zur Ergänzung eingesetzte Wasser kann Frischwasser sein oder Wasser, welches bei der Aufarbeitung der bei dem erfindungsgemäßen Verfahren anfallenden Ströme anfällt.

Der abgetrennte Produktstrom (Rohcarbonsäure) kann noch geringe Katalysatormengen enthalten. Um die Katalysatorverluste klein zu halten und ein möglichst Katalysator-freies Produkt zu erhalten, kann es vorteilhaft sein, den Produktstrom mit Wasser zu extrahieren. Dies kann in technisch üblichen Apparaturen zweckmäßig im Gegenstrom erfolgen. Die Extraktion kann bei Temperaturen von 0 bis 200 °C durchgeführt werden. In den meisten Fällen ist es zweckmäßig, die Extraktion im Temperaturbereich von 15 bis 95 °C bei Normaldruck durchzuführen.

Das bei der Wäsche des Rohproduktes anfallende wässrige Extrakt kann in die aus dem Trennbehälter zum Reaktor zurückgeführte Katalysatorphase gegeben (gepumpt) werden. Dabei ist darauf zu achten, dass nur so viel Wasser in die Katalysatorphase eingespeist wird, wie bei der Hydrocarboxylierung verbraucht wird. Enthält das Extrakt mehr Wasser als zum Ausgleich der Wasserbilanz notwendig ist, bestehen im Prinzip mehrere Möglichkeiten. Die erste besteht darin, einen Teil des Extraktes auszuschleusen. Dies kann aber zu Katalysatorverlusten führen. Die zweite, bevorzugte Möglichkeit besteht darin, vor der Rückführung einen Teil des Wassers zu entfernen, beispielsweise durch Destillation oder durch Membrantrennung. Enthält das Extrakt zu wenig Wasser wird zusätzlich Frischwasser in die Katalysatorlösung eingespeist.

Das gewaschene Rohprodukt kann nach bekannten Verfahren, beispielsweise durch Destillation, zu den, überwiegend tertiären, Carbonsäuren aufgearbeitet werden.

Die bei der Reaktion entstehende Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen. Art und Größe sowie Einbaustandort der verwendeten Wärmetauscher sind von diesen Parametern abhängig. Die Wärmetauscher können insbesondere in der Rückführung der Phasen, insbesondere der kontinuierlichen Phase angeordnet sein.

Die erfindungsgemäß hergestellten Carbonsäuren können beispielsweise zur Herstellung von Sikkativen, Peroxiester oder Schmierstoffen verwendet werden. Weiterhin können sie zur Herstellung von ungesättigten Estern, beispielsweise zu Vinylestern verwendet werden, die als Comonomere Verwendung finden.

Ein Blockschema einer Anlage, in der das erfindungsgemäße Verfahren durchgeführt werden kann, ist in Fig. 1 dargestellt, wobei das erfindungsgemäße Verfahren nicht auf die dort abgebildete Ausführungsform beschränkt sein soll.

Auf den Kopf des Hydrocarboxylierungsreaktors 1 wird die Katalysatorphase 8 zusammen mit Olefin 2, Kohlenmonoxid-haltigem Gas 3 und Wasser 4 eingespeist. Das als Reaktionsgemisch aus dem Reaktor 1 erhaltene Gemisch 5 wird im Absitzbehälter 6 in Restgas 7, Katalysatorphase 8 und Produktphase 9 getrennt. Die Katalysatorphase wird, gegebenenfalls nach Ausschleusung einer kleinen Teilmenge und deren Ersatz durch frischen Katalysator, mit Hilfe einer Umlaufpumpe in den Reaktor 1 zurückgeführt. Das Restgas 7 wird im Kühler 10 gekühlt und gegebenenfalls anfallendes Kondensat durch eine nicht dargestellten Leitung in den Behälter 6 geleitet. Die abgetrennte Produktphase wird im Gefäß 12 mit Wasser 13 gewaschen. Das Waschwasser 15 kann teilweise das Frischwasser 4 ersetzen. Die gewaschene Produktphase kann nach bekannten Verfahren aufgearbeitet werden.

Die nachfolgenden Beispiele sollen die Erfindung näher erläutern, ohne ihre Anwendungsbreite zu begrenzen, die sich aus den Patentansprüchen ergibt.

**Versuchsaufbau:**

Die Versuche wurden in einer kontinuierlichen Versuchsapparatur durchgeführt, deren Aufbau weitgehend dem Blockschema (Figur 1) entspricht.

In einem Reaktor (Blasensäule oder Strömungsrohr) (1) wurden Katalysatorphase (8), Olefm (2) (verdünnt mit n-Heptan oder Cyclohexan) und Synthesegas (3) zur Reaktion gebracht. Aus dem Reaktor gelangte das Drei-Phasen-Gemisch zum Phasentrenner (6) (Behälter mit eingebautem Wehr). Die schwere flüssige Katalysatorphase (8) setzte sich ab und ging über eine Kreiselpumpe zurück zum Reaktor (1). Die leichtere organische Phase (9) lief über das Wehr in eine zweite Kammer des Phasentrenners und wurde standgeregelt abgezogen. Der im Synthesegas (3) enthaltene Wasserstoff, der nicht an der Reaktion teilnahm, sowie nicht umgesetztes Synthesegas gasten in (6) aus und wurden einem in Figur 1 nicht abgebildeten Abgaswäscher G zugeführt. Dort wurden flüchtige Carbonsäuren und Katalysatorbestandteile mit Wasser ausgewaschen. Mit dem anfallenden Waschwasser (13) wurde das Rohprodukt (9) im Extraktor (12) Katalysator-frei gewaschen und der dabei entstandene Extrakt in die Katalysatorphase gepumpt.

Als Einsatzolefin wurden Octen-Gemische, hergestellt nach dem OCTOL-Prozess der OXENO Olefinchemie GmbH durch Oligomerisierung von n-Buten oder Buten-Destillationsschnitten, eingesetzt. In den Versuchen 1 bis 28 hatte das eingesetzte Octengemisch (Dibuten) folgende Zusammensetzung: 13 % n-Octene, 62 % 3-Methylheptene, 24 % 3,4-Dimethylhexene, 1 % andere $C_8$-Olefine

Als CO-Quelle wurde technisches Synthesegas mit 50-55 Vol.-% CO (Rest Wasserstoff) oder reines Kohlenmonoxid eingesetzt.

Als Katalysator wurde ein Komplexe aus $BF_3$, HF, $H_2O$ und $Cu^+$, der der folgenden Formel genügt, eingesetzt:

$$CU_aH_{(1-a)}[BF_{(3+b)}(OH)_{(1-b)}]\ [H_2O]_n$$

mit

a = 0,05 bis 0,15
b = 0 bis 0,5 und
n = 0,5 bis 1

In der Reaktion verbrauchtes Wasser wurde mit vollentsalztem Wasser ergänzt.

**Beispiel 1 (Vergleichsbeispiel)**

Als Reaktor wurde eine Blasensäule eingesetzt. Die Katalysatorphase wurde am Fuß der Säule eingepumpt. Dibuten und Synthesegas wurden über getrennte Fritten ebenfalls am Fuß der Säule eingespeist. Der Kopf des Reaktors war als Ausgaser für das Restgas erweitert, die flüssigen Bestandteile liefen zu einem Phasentrenner (Settler)

über.

| Maße des Reaktors: | |
|---|---|
| Länge (Flüssigkeitssäule bis zum Überlauf) | 4400 mm |
| Innendurchmesser | 209 mm |
| Volumen der Flüssigkeitssäule ca. | 150 Liter |

Während des Versuches schwankte die Katalysatorzusammensetzung in folgenden Grenzen:

a = 0,09 bis 0,10 Mol/Mol (Cu$^+$ bezogen auf Bor)
b = 0,1 bis 0,2 Mol/Mol (das heißt, Fluor bezogen auf Bor = 3,1 bis 3,2 Mol/Mol)
n = 0,8 bis 0,9 Mol/Mol

| | |
|---|---|
| Druck (nach Reaktor) | 1,5 MPa Überdruck |
| Temperatur (nach Reaktor) | 30 bis 35 °C |

Die besten Ergebnisse wurden mit folgenden Daten erreicht:

| | |
|---|---|
| Katalysatorkreislauf | 2,0 t/h (etwa 1,2 m/h bei 20 °C) |
| Synthesegas | 10 $m^3$/h bei Normbedingungen (0 °C, 0,1013 MPa) |
| Olefinzugang | 35 kg/h |

Der Umsatz schwankte im Bereich von 50 bis 60 Mol-%, die Selektivität im Bereich 78 bis 81 %. Der Rest des Produktstromes bestand im Wesentlichen aus dimerisiertem Dibuten neben Spuren kürzer- und längerkettiger Säuren, $C_8$-Akoholen und Estern von $C_8$-Alkoholen mit den gebildeten Carbonsäuren. Über 99 % der erhaltenen Carbonsäuren waren tertiär verzweigt.
Die Raum-Zeit-Ausbeute an Summe der Nonansäuren lag bei 0,14 bis 0,15 t/($m^3$∗h) (bezogen auf den flüssigkeitsgefüllten Teil der Blasensäule).
Der berechnete Belastungsfaktor B lag bei Verwendung einer Blasensäule ohne Füllkörper im Bereich von $4*10^{-6}$ bis $5*10^{-6}$.

**Beispiele 2 bis 11**

In einer verkleinerten Laborapparatur nach dem zuvor beschriebenen Prinzip wurden Versuche in einer Gleichstrom-Füllkörper-Kolonne durchgeführt. Als Reaktor diente ein Rohr DN 50 (Innendurchmesser 53 mm) mit einer Länge von 1000 mm, das über eine Länge von 950 mm mit Maschendrahtringen (Vereinigte Füllkörper Fabriken, VFF; 4 x 4 mm mit Steg, berechneter hydraulischer Durchmesser $d_H$ = 1,913 mm, freies Volumen 91,5 %) gefüllt war. Als Reaktorvolumen wurde das Leerrohrvolumen der Füllkörperschicht verwendet, es betrug 2,10 Liter. Katalysator, Synthesegas und organische Eduktphase (Olefin in Cyclohexan, je 50 Massen-%) wurden unmittelbar vor dem Reaktor ohne weitere Mischeinrichtungen zusammengeführt. Der Reaktor wurde von oben nach unten durchströmt.
Das Synthesegas wurde mengengeregelt eindosiert, das Eduktgemisch über eine Pumpe eingefahren (Mengenbestimmung aus der Volumenabnahme der Vorlage) und der Katalysatorkreislauf über eine Kreiselpumpe eingestellt. Die angegebene Katalysatorkonzentration war der Mittelwert aus Analysen zu Beginn, während und nach den Versuchen. Umsatz und Selektivität wurden durch gaschromatographische Analyse des über 3 Stunden konstanter Fahrweise gesammelten Rohprodukts ermittelt.
Die Querschnittsbelastung des Reaktors wurde bei 3 verschiedenen Phasenverhältnissen Katalysatorphase/Eduktphase variiert.
Die nachfolgenden Ergebnisse zeigen, dass sich bei steigender Querschnittsbelastung des Reaktors trotz sinkender Verweilzeit der Umsatz und die Selektivität nur wenig ändern. Dementsprechend nimmt die Raum-Zeit-Ausbeute fast linear mit der Querschnittsbelastung bei gleichen Phasenverhältnissen zu. Bei Versuch 5 wurde eine Raum-Zeit-Ausbeute von 0,272 t/$m^3$/h bei einer Selektivität von 95,2 Mol-% und einem Olefinumsatz von 94,3 % erreicht. Bei Versuch 9 wurde mit einer Raum-Zeit-Ausbeute von 0,441 t/($m^3$∗h) etwa der dreifach höhere Wert als mit der Blasensäule in Beispiel 1 erreicht.

Tabelle 1:

| Versuchsdaten der Beispiele 2 bis 11 | | | | | | | | | | |
|---|---|---|---|---|---|---|---|---|---|---|
| Versuch | 2 | 3 | 4 | 5 | 6 | 7 | 8 | 9 | 10 | 11 |
| Druck (MPa) | 1,5 | 1,5 | 1,5 | 1,5 | 1,5 | 1,5 | 1,5 | 1,5 | 1,5 | 1,5 |
| Temperatur (°C) | 31 | 31 | 32 | 33 | 34 | 32 | 35 | 34 | 32 | 31 |
| | | | | | | | | | | |
| Katalysator (kg/h) | 44 | 98 | 146 | 195 | 49 | 98 | 146 | 195 | 49 | 98 |
| Katalysator (l/h) | 27 | 61 | 91 | 121 | 30 | 61 | 91 | 122 | 30 | 61 |
| WasserBor (Mol/Mol) | 1,73 | 1,74 | 1,8 | 1,7 | 1,78 | 1,78 | 1,87 | 1,77 | 1,78 | 1,99 |
| FluorBor (Mol/Mol) | 3,1 | 2,92 | 3,19 | 2,91 | 3,16 | 3,16 | 3,24 | 2,88 | 3,16 | 3,31 |
| Cu+Bor (Mol/Mol) | 0,01 | 0,01 | 0,009 | 0,01 | 0,01 | 0,01 | 0,011 | 0,009 | 0,01 | 0,012 |
| | | | | | | | | | | |
| Synthesegas (kg/h) | 0,068 | 0,138 | 0,208 | 0,272 | 0,141 | 0,277 | 0,415 | 0,545 | 0,277 | 0,554 |
| Synthesegas (l/h) | 7 | 14 | 21 | 28 | 14 | 28 | 42 | 56 | 28 | 56 |
| CO-Anteil (Vol.-%) | 51 | 52 | 52 | 51 | 53 | 52 | 52 | 51 | 52 | 52 |
| | | | | | | | | | | |
| Edukt (kg/h) | 0,22 | 0,45 | 0,71 | 0,90 | 0,47 | 0,82 | 1,38 | 1,73 | 0,89 | 1,76 |
| Edukt (l/h) | 0,3 | 0,62 | 0,98 | 1,25 | 0,65 | 1,14 | 1,91 | 2,40 | 1,24 | 2,43 |
| Olefmanteil (Massen-%) | 50 | 50 | 50 | 50 | 50 | 50 | 50 | 50 | 50 | 50 |
| | | | | | | | | | | |
| Verweilzeit (s) | 218 | 100 | 67 | 50 | 168 | 84 | 56 | 42 | 127 | 64 |
| | | | | | | | | | | |
| Massenverhältnis von Katalysator zu Synthesegas) | 644 | 704 | 704 | 716 | 346 | 352 | 352 | 358 | 176 | 176 |
| Massenverhältnis von Katalysator zu Edukt) | 399,6 | 436,7 | 412 | 433,2 | 208,1 | 237,9 | 212,6 | 224,8 | 109,2 | 111 |
| Massenverhältnis von Synthesegas zu Edukt) | 0,62 | 0,62 | 0,59 | 0,60 | 0,60 | 0,68 | 0,60 | 0,63 | 0,62 | 0,63 |
| | | | | | | | | | | |
| Umsatz (%) | 99,6 | 88,5 | 93,7 | 94,3 | 82,6 | 89,0 | 90,2 | 83,9 | 77,3 | 42,7 |
| Selektivität (%) | 95,7 | 94,7 | 95,2 | 95,2 | 89,4 | 87,9 | 92,5 | 90,1 | 81,4 | 79,1 |
| RZA ($t/m^3*h$) | 0,068 | 0,126 | 0,213 | 0,272 | 0,116 | 0,216 | 0,386 | 0,441 | 0,189 | 0,200 |
| | | | | | | | | | | |
| Belastungsfaktor B | 0,043 | 0,097 | 0,148 | 0,201 | 0,060 | 0,124 | 0,186 | 0,260 | 0,089 | 0,187 |

**Beispiele 12 bis 16**

Um in der zur Verfügung stehenden Apparatur höhere Querschnittsbelastungen testen zu können, wurde der Reaktor durch Einziehen eines Teflonhemdes verkleinert. Der Reaktor hatte danach einen Innendurchmesser von nur 30 mm, die Füllkörperschicht betrug wieder 950 mm. Als Füllkörper wurden wieder die Maschendrahtringe 4x4 mm

wie in den Beispielen 2 bis 11 verwendet.

Zwar zeigten die Versuche einen gewissen Einfluss der Katalysatorzusammensetzung auf Selektivität und Umsatz, doch war eindeutig zu erkennen, dass die Raum-Zeit-Ausbeute weiter gesteigert wurde. Bei Versuch 16 wurde mit 1,375 t/($m^3$*h) eine fast 10-fache Steigerung gegenüber dem Vergleichsversuch in der Blasensäule (Beispiel 1) erzielt!

Tabelle 2:

| Versuchsdaten der Beispiele 12 bis 16 | | | | | |
|---|---|---|---|---|---|
| Versuch | 12 | 13 | 14 | 15 | 16 |
| Druck (MPa) | 1,5 | 1,5 | 1,5 | 1,5 | 1,5 |
| Temperatur (°C) | 31 | 35 | 37 | 42 | 43 |
| | | | | | |
| Katalysator (kg/h) | 137 | 187 | 172 | 171 | 171 |
| Katalysator (l/h) | 85 | 117 | 108 | 107 | 107 |
| WasserBor (Mol/Mol) | 1,96 | 2,08 | 2,05 | 1,92 | 1,81 |
| FluorBor (Mol/Mol) | 3 | 3,18 | 3,20 | 3,24 | 3,34 |
| Cu+Bor (Mol/Mol) | 0,008 | 0,015 | 0,018 | 0,018 | 0,018 |
| | | | | | |
| Synthesegas (kg/h) | 0,199 | 0,265 | 0,485 | 0,485 | 0,485 |
| Synthesegas (Nl/h) | 200 | 270 | 500 | 500 | 500 |
| CO-Anteil (Vol.-%) | 52 | 52 | 52 | 52 | 52 |
| | | | | | |
| Edukt (kg/h) | 0,64 | 0,861 | 1,52 | 1,46 | 1,49 |
| Edukt (l/h) | 0,881 | 1,191 | 2,121 | 2,053 | 2,096 |
| Olefmanteil (Massen-%) | 50 | 50 | 50 | 50 | 50 |
| | | | | | |
| Verweilzeit (s) | 23 | 17 | 15 | 15 | 15 |
| | | | | | |
| Massenverhältnis von Katalysator zu Synthesegas) | 687 | 705 | 355 | 352 | 352 |
| Massenverhältnis von Katalysator zu Edukt) | 428,7 | 436,4 | 226,3 | 233,1 | 228,5 |
| Massenverhältnis von Synthesegas zu Edukt) | 0,62 | 0,62 | 0,64 | 0,66 | 0,65 |
| | | | | | |
| Umsatz (%) | 74,5 | 84,7 | 81,0 | 94,7 | 97,6 |
| Selektivität (%) | 88,0 | 88,1 | 86,3 | 92,9 | 89,8 |
| RZA (t/$m^3$*h) | 0,439 | 0,671 | 1,118 | 1,353 | 1,375 |
| | | | | | |
| Belastungsfaktor B | 0,522 | 0,740 | 0,855 | 0,812 | 0,806 |

**Beispiele 17 bis 24**

In die in Beispiel 2 verwendete Versuchsanlage wurde ein noch kleinerer Reaktor eingebaut: Innendurchmesser 11 mm, Länge 900 mm (Füllkörperzone).

Die Versuchsergebnisse belegen, dass trotz der extrem kurzen Verweilzeit nach wie vor hohe Umsätze erreicht wurden, die Raum-Zeit-Ausbeuten stiegen bis auf fast 17 b($m^3$*h). Dies war eine Erhöhung gegenüber der

Umsetzung in der Blasensäule (Beispiel 1) um über zwei Zehnerpotenzen.

Tabelle 3:

| Versuchsdaten der Beispiele 17 bis 24 | | | | | | | | |
|---|---|---|---|---|---|---|---|---|
| Versuch | 17 | 18 | 19 | 20 | 21 | 22 | 23 | 24 |
| Druck (MPa) | 0,8 | 0,8 | 0,8 | 1,5 | 1,5 | 1,5 | 1,5 | 1,5 |
| Temperatur (°C) | 31 | 34 | 32 | 30 | 30 | 31 | 34 | 40 |
| | | | | | | | | |
| Katalysator (kg/h) | 73 | 146 | 219 | 146 | 146 | 239 | 81 | 229 |
| Katalysator (l/h) | 45 | 91 | 137 | 91 | 91 | 149 | 51 | 144 |
| WasserBor (Mol/Mol) | 1,88 | 2,13 | 2,09 | 2,01 | 1,91 | 1,75 | 1,92 | 1,95 |
| FluorBor (Mol/Mol) | 3,44 | 3,64 | 3,66 | 3,45 | 3,37 | 3,1 | 3,53 | 3,50 |
| Cu+Bor (Mol/Mol) | 0,02 | 0,019 | 0,02 | 0,02 | 0,017 | 0,015 | 0,017 | 0,006 |
| | | | | | | | | |
| (Synthesegas) (kg/h) | 0,187 | 0,375 | 0,562 | 0,208 | 0,208 | 0,340 | 0,340 | 0,692 |
| (Synthesegas) (Nl/h) | 19 | 37 | 56 | 21 | 21 | 35 | 35 | 72 |
| CO-Anteil (Vol.-%) | 100 | 100 | 100 | 52 | 52 | 51 | 51 | 52 |
| | | | | | | | | |
| Edukt (kg/h) | 0,34 | 0,67 | 0,97 | 0,69 | 0,66 | 1,05 | 1,05 | 2,14 |
| Edukt (l/h) | 0,466 | 0,935 | 1,349 | 0,951 | 0,911 | 1,459 | 1,459 | 3,000 |
| Olefmanteil (Massen-%) | 50 | 50 | 50 | 50 | 50 | 50 | 50 | 50 |
| | | | | | | | | |
| Verweilzeit (s) | 5 | 3 | 2 | 3 | 3 | 2 | 4 | 1 |
| | | | | | | | | |
| Massenverhältnis von Katalysator zu Synthesegas) | 390 | 390 | 390 | 704 | 704 | 702 | 239 | 331 |
| Massenverhältnis von Katalysator zu Edukt) | 434,4 | 434,4 | 450,7 | 425,1 | 444,0 | 453,3 | 155,3 | 213,7 |
| Massenverhältnis von Synthesegas zu Edukt) | 1,11 | 1,11 | 1,16 | 0,60 | 0,63 | 0,65 | 0,65 | 0,65 |
| | | | | | | | | |
| Umsatz (%) | 95,3 | 96,6 | 96,8 | 94,9 | 95,4 | 98,3 | 95,1 | 97,2 |
| Selektivität (%) | 86,9 | 93,0 | 89,9 | 89,8 | 88,9 | 91,1 | 85,4 | 93,3 |
| RZA ($t/m^{3*}h$) | 2,30 | 4,989 | 6,987 | 4,836 | 4,608 | 7,784 | 7,011 | 16,040 |
| | | | | | | | | |
| Belastungsfaktor B | 2,657 | 7,438 | 14,603 | 6,041 | 6,039 | 13,518 | 4,013 | 16,851 |

**Beispiele 25 bis 28**

In dieser Versuchsreihe wurde mit reinem Kohlenmonoxid (> 99 %) gearbeitet und der Einfluss des CO-Drucks untersucht. Daneben wurden am Reaktor Manometer installiert, die die Messung des Differenzdruckes über den Reaktor gestatteten. Weiterhin wurden Temperaturmessfühler eingebaut, um die Temperaturerhöhung im Reaktor zu messen. Der Reaktor hatte eine Länge von 1000 mm (Füllkörperzone), einen Innendurchmesser von 16,0 mm und war vollständig mit Armaflex-Schläuchen gegen Wärmeabstrahlung isoliert, so dass eine annähernd adiabatische Fahrweise realisiert wurde.

Die Messungen belegten, dass bei hoher Raum-Zeit-Ausbeute (10 bis 11 t/(m$^3$*h)) mit reinem Kohlenmonoxid und steigendem Druck die Selektivität verbessert werden kann. Bei Versuch 28 wurde praktisch vollständiger Umsatz und eine Selektivität von fast 97 % erreicht. Die Temperaturerhöhung über den Reaktor war mit 2,8 °C immer noch so niedrig, dass keine Wärmeabfuhrprobleme auftraten. Auch der Differenzdruck von 0,07 MPa war technisch kein Problem.

Tabelle 4:

| Versuchsdaten der Beispiele 25 bis 28 | | | | |
|---|---|---|---|---|
| Versuch | 25 | 26 | 27 | 28 |
| Druck (MPa) | 0,5 | 1,0 | 1,5 | 2,0 |
| Temperatur (°C) | 40 | 42 | 43 | 44 |
| | | | | |
| Katalysator (kg/h) | 241 | 244 | 244 | 244 |
| Katalysator (l/h) | 151 | 153 | 153 | 153 |
| WasserBor (Mol/Mol) | 1,48 | 1,56 | 1,57 | 1,55 |
| FluorBor (Mol/Mol) | 3,23 | 3,26 | 3,31 | 3,44 |
| Cu+Bor (Mol/Mol) | 0,007 | 0,007 | 0,007 | 0,007 |
| | | | | |
| Synthesegas (kg/h) | 0,999 | 0,999 | 0,999 | 0,999 |
| (Synthesegas) (Nl/h) | 153 | 153 | 153 | 153 |
| CO-Anteil (Vol.-%) | 100 | 100 | 100 | 100 |
| | | | | |
| Edukt (kg/h) | 2,93 | 2,93 | 2,93 | 2,93 |
| Edukt (l/h) | 4,095 | 4,105 | 4,110 | 4,115 |
| Olefmanteil (Massen-%) | 50 | 50 | 50 | 50 |
| | | | | |
| Verweilzeit (s) | 2 | 3 | 3 | 4 |
| | | | | |
| Massenverhältnis von Katalysator zu Synthesegas) | 241 | 244 | 244 | 244 |
| Massenverhältnis von Katalysator zu Edukt) | 164,3 | 166,5 | 166,5 | 166,5 |
| Massenverhältnis von Synthesegas zu Edukt) | 0,68 | 0,68 | 0,68 | 0,68 |
| | | | | |
| Umsatz (%) | 99,6 | 99,6 | 99,6 | 99,6 |
| Selektivität (%) | 92,2 | 95,3 | 96,2 | 96,8 |
| RZA (t/m$^3$*h) | 9,432 | 9,749 | 9,832 | 9,903 |
| | | | | |
| Belastungsfaktor B | 12,595 | 8,833 | 7,420 | 6,677 |

**Beispiele 29-30**

Die in den Beispielen 2 bis 28 verwendete Anlage wurde zur Durchführung des erfindungsgemäßen Verfahrens mit einer anderen Reaktionsdimension und anderen Füllkörpern verwendet.

Als Reaktor wurde ein von oben nach unten durchströmtes Rohr DN 40 (Innendurchmesser 44.3 mm) von

2000 mm Länge verwendet. Das Rohr war auf einer Länge von 1920 mm mit Sulzer-Mischern vom Typ SMV 8 bestückt. Das Leerrohrvolumen bezogen auf die Füllkörperlänge betrug 2,96 Liter.

Als Edukt wurde ein Olefingemisch von im Wesentlichen innenständigen verzweigten Olefinen eingesetzt, das nach gaschromatographischer Analyse folgende Zusammensetzung hatte:

| | |
|---|---|
| 2,4-Dimethyl-Hexen | 2,54 Vol.-% |
| 3,3-Dimethyl-Hexen | 1,02 Vol.-% |
| 2,3-Dimethyl-Hexen | 1,60 Vol.-% |
| 3,4-Dimethyl-Hexen | 63,54 Vol.-% |
| 3-Methyl-Hepten | 29,13 Vol.-% |
| Sonstige Octene | 2,14 Vol.-% |

Das Olefmgemisch wurde zu Beginn des Versuchs mit n-Heptan gemischt und in einem Behälter vorgelegt. Das Rohprodukt wurde nach Phasentrennung mit dem zur Reaktion benötigten Wasser katalysatorfrei gewaschen, die Waschwässer gingen zurück zum Reaktor. Das gewaschene Rohprodukt gelangte zu einer Destillationskolonne, in der das Lösungsmittel und nicht umgesetztes Olefm über Kopf abgetrennt wurden. Lösungsmittel und Restolefm wurden zu dem Vorlagebehälter für das Einsatzgemisch zurückgeführt. Verbrauchtes Olefm wurde standgeregelt in den Vorlagebehälter nachgespeist.

Die Anlage wurde kontinuierlich betrieben, wobei nur gelegentlich kleine Mengen Lösungsmittel ergänzt werden mussten, die bei der Destillation durch ungenügende Kondensation verloren gingen. Es konnten ein Gemisch tertiärer Nonansäuren mit einer Reinheit von 99.8 % und in einer Selektivität von 93 bis 96 % hergestellt werden.

Tabelle 5.

| Versuchsdaten der Beispiele 29 und 30 | | |
|---|---|---|
| Versuch | 29 | 30 |
| Druck (MPa) | 1,5 | 1,5 |
| Temperatur (°C) | 22 | 27 |
| | | |
| Katalysator (kg/h) | 5,999 | 5,999 |
| Katalysator (l/h) | 3,643 | 3,659 |
| WasserBor (Mol/Mol) | 1,49 | 1,53 |
| FluorBor (Mol/Mol) | 3,36 | 3,33 |
| Cu+Bor (Mol/Mol) | 0,0127 | 0,0136 |
| | | |
| Synthesegas (kg/h) | 10,313 | 9,669 |
| Synthesegas (Nl/h) | 1001 | 934 |
| CO-Anteil (Vol.-%) | 52,3 | 53,6 |
| | | |
| Edukt (kg/h) | 49,04 | 43,92 |
| Edukt (l/h) | 67,16 | 60,49 |
| Olefmanteil (Massen-%) | 40 | 55 |
| | | |
| Verweilzeit (s) | 2 | 2 |
| | | |
| Massenverhältnis von Katalysator zu Synthesegas) | 582 | 620 |
| Massenverhältnis von Katalysator zu Edukt) | 305,8 | 248,4 |

Tabelle 5. (fortgesetzt)

| Versuchsdaten der Beispiele 29 und 30 | | |
|---|---|---|
| Versuch | 29 | 30 |
| Massenverhältnis von Synthesegas zu Edukt) | 0,53 | 0,40 |
| | | |
| Umsatz (%) | 99,2 | 97,5 |
| Selektivität (%) | 94,3 | 96,9 |
| RZA ($t/m^3*h$) | 8,747 | 10,766 |
| | | |
| Belastungsfaktor B | 3,606 | 3,511 |

**Patentansprüche**

**1.** Katalytisches Mehrphasenreaktionsverfahren, bei dem zumindest drei Edukte eingesetzt werden,
**dadurch gekennzeichnet,**
**dass** das Verfahren in zumindest einem Rohrreaktor durchgeführt wird, wobei der Katalysator in einer kontinuierlichen Phase enthalten ist, zumindest ein Edukt in dieser kontinuierlichen Phase enthalten ist, und zumindest zwei Edukte in der kontinuierlichen Phase dispergiert enthalten sind und der Belastungsfaktor B des Rohrreaktors gleich oder größer 0,2 ist.

**2.** Verfahren nach Anspruch 1,
**dadurch gekennzeichnet,**
**dass** die zumindest zwei Edukte, die in der kontinuierlichen Phase dispergiert enthalten sind, in zwei unterschiedlichen dispersen Phasen in der kontinuierlichen Phase enthalten sind.

**3.** Verfahren nach Anspruch 1 oder 2,
**dadurch gekennzeichnet,**
**dass** der Belastungsfaktor größer oder gleich 0,9 ist.

**4.** Verfahren nach Anspruch 3,
**dadurch gekennzeichnet,**
**dass** der Belastungsfaktor größer oder gleich 1,0 ist.

**5.** Verfahren nach einem der Ansprüche 1 bis 4,
**dadurch gekennzeichnet,**
**dass** das Massenverhältnis der kontinuierlichen Phase zu den dispersen Phasen größer 2 ist.

**6.** Verfahren nach einem der Ansprüche 1 bis 5,
**dadurch gekennzeichnet,**
**dass** die kontinuierliche Phase eine Strahldüse vor dem Rohrreaktor antreibt.

**7.** Verfahren nach einem der Ansprüche 1 bis 6,
**dadurch gekennzeichnet,**
**dass** mindestens ein Edukt durch die von der kontinuierlichen Phase in den Rohrreaktor eingebrachte Energie dispergiert wird.

**8.** Verfahren nach einem der Ansprüche 1 bis 7,
**dadurch gekennzeichnet,**
**dass** in der Mehrphasenreaktion zumindest ein Olefin zu einer Carbonsäure hydrocarboxyliert wird.

**9.** Verfahren nach Anspruch 8,
**dadurch gekennzeichnet,**

**dass** die bei der Hydrocarboxylierung eingesetzten Edukte zumindest Wasser, Kohlenstoffmonoxid und zumindest ein Olefin mit 4 bis 25 Kohlenstoffatome umfassen, wobei das Wasser als Edukt in der kontinuierlichen Phase, und das Kohlenstoffmonoxid und das zumindest eine Olefin dispergiert in der kontinuierlichen Phase vorliegen.

**10.** Verfahren nach Anspruch 9,
**dadurch gekennzeichnet,**
**dass** Kohlenstoffmonoxid und zumindest ein Olefin als eine gasförmige Phase dispergiert in der kontinuierlichen Phase vorliegen.

**11.** Verfahren nach Anspruch 9,
**dadurch gekennzeichnet,**
**dass** Kohlenstoffmonoxid und zumindest eine Olefin in zwei unterschiedlichen dispergierten Phasen vorliegen.

**12.** Verfahren nach zumindest einem der Ansprüche 8 bis 11,
**dadurch gekennzeichnet,**
**dass** die Katalysatorphase bei der Hydrocarboxylierung eine komplexe Verbindung der Formel

$$Cu_aH_{(1-a)}[BF_{(3+b)}(OH)_{(1-b)}]\ [H_2O]_n$$

mit a = 0,05 bis 0,15
b = 0,00 bis 0,50 und
n = 0,50 bis 1,00
aufweist.

**13.** Verfahren nach zumindest einem der Ansprüche 8 bis 12,
**dadurch gekennzeichnet,**
**dass** die Hydrocarboxylierung bei einer Temperatur von 10 bis 150 °C durchgeführt wird.

**14.** Verfahren nach zumindest einem der Ansprüche 9 bis 13,
**dadurch gekennzeichnet,**
**dass** das Verfahren bei einem molaren Verhältnis von Kohlenstoffmonoxid zu Olefin bzw. Olefinen von 2 zu 1 bis 5 zu 1 durchgeführt wird.

**15.** Verfahren nach zumindest einem der Ansprüche 9 bis 14,
**dadurch gekennzeichnet,**
**dass** das Verfahren bei einem Massenverhältnis von Katalysatorphase zu Olefinphase von 10 zu 1 bis 1000 zu 1 durchgeführt wird.

**16.** Verfahren nach zumindest einem der Ansprüche 9 bis 15,
**dadurch gekennzeichnet,**
**dass** das Verfahren bei einem Massenverhältnis von Katalysatorphase zu Kohlenstoffmonoxidphase von 50 zu 1 bis 1000 zu 1 durchgeführt wird.

**17.** Verfahren nach zumindest einem der Ansprüche 9 bis 16,
**dadurch gekennzeichnet,**
**dass** als Olefine $C_8$-Olefine eingesetzt werden.

**18.** Verwendung von Carbonsäuren, hergestellt nach einem Verfahren gemäß zumindest einem der Ansprüche 8 bis 17, zur Herstellung von Schmiermitteln, Sikkativen, Peroxiestern oder ungesättigten Estern, insbesondere Vinylestern.

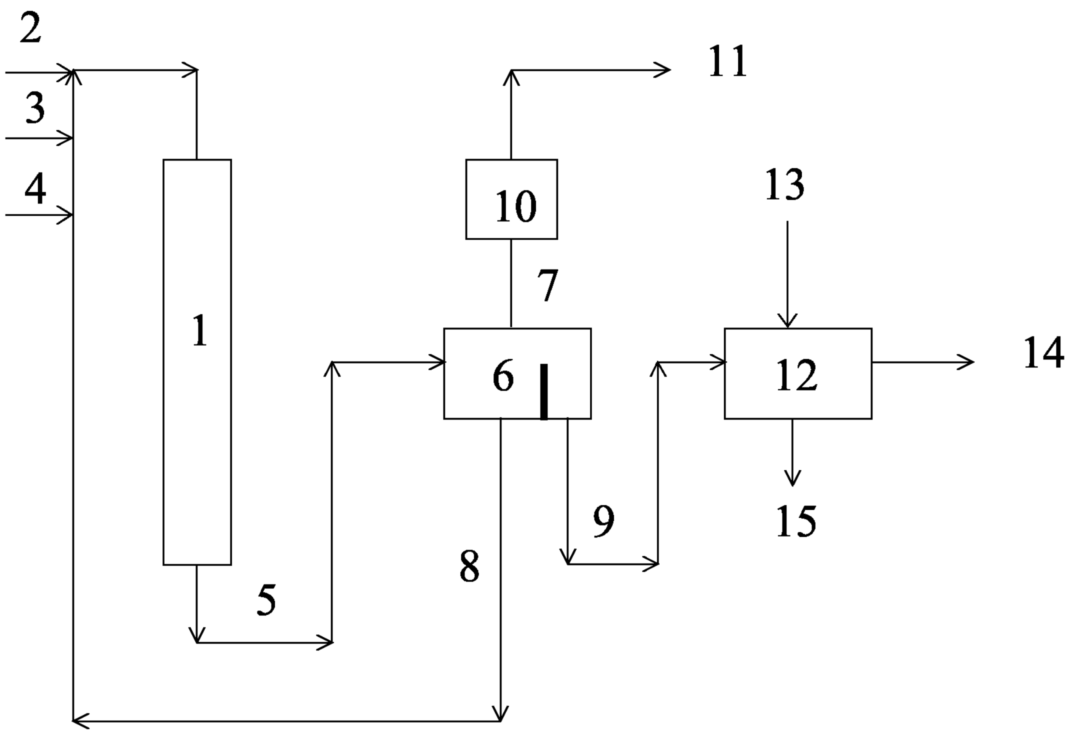


Fig. 1

Europäisches Patentamt

# EUROPÄISCHER RECHERCHENBERICHT

Nummer der Anmeldung
EP 05 10 0167

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 6 492 564 B1 (WIESE KLAUS-DIETHER ET AL) 10. Dezember 2002 (2002-12-10)<br>* Ansprüche 1,2,6-12 *<br>----- | 1-7 | B01J19/24 |
| X | EP 1 057 525 A (OXENO OLEFINCHEMIE GMBH) 6. Dezember 2000 (2000-12-06)<br>* das ganze Dokument *<br>----- | 1-7 | |
| X | EP 1 106 594 A (OXENO OLEFINCHEMIE GMBH) 13. Juni 2001 (2001-06-13)<br>* Ansprüche 1-14 *<br>----- | 1-7 | |
| X | EP 1 106 596 A (OXENO OLEFINCHEMIE GMBH) 13. Juni 2001 (2001-06-13)<br>* Ansprüche 1-13 *<br>----- | 1-7 | |

RECHERCHIERTE SACHGEBIETE (Int.Cl.7)
B01J

Der vorliegende Recherchenbericht wurde für alle Patentansprüche erstellt

2

| Recherchenort | Abschlußdatum der Recherche | Prüfer |
|---|---|---|
| Den Haag | 8. Juli 2005 | Vlassis, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 10 0167

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-07-2005

| Im Recherchenbericht angeführtes Patentdokument | Datum der Veröffentlichung | Mitglied(er) der Patentfamilie | Datum der Veröffentlichung |
|---|---|---|---|
| US 6492564 B1 | 10-12-2002 | DE 19925384 A1 | 07-12-2000 |
| | | BR 0002178 A | 02-01-2001 |
| | | CA 2310516 A1 | 02-12-2000 |
| | | CN 1276364 A | 13-12-2000 |
| | | EP 1057524 A2 | 06-12-2000 |
| | | ID 26185 A | 07-12-2000 |
| | | JP 2001026566 A | 30-01-2001 |
| | | MX PA00001215 A | 24-04-2002 |
| | | PL 340362 A1 | 04-12-2000 |
| | | SG 86401 A1 | 19-02-2002 |
| | | TW 537930 B | 21-06-2003 |
| | | ZA 200002740 A | 11-12-2000 |
| EP 1057525 A | 06-12-2000 | DE 19925385 A1 | 07-12-2000 |
| | | BR 0002555 A | 02-01-2001 |
| | | CA 2310512 A1 | 02-12-2000 |
| | | CN 1290684 A ,C | 11-04-2001 |
| | | EP 1057525 A2 | 06-12-2000 |
| | | JP 2001019660 A | 23-01-2001 |
| | | MX PA00005432 A | 04-06-2002 |
| | | PL 340447 A1 | 04-12-2000 |
| | | SG 85706 A1 | 15-01-2002 |
| | | TW 523423 B | 11-03-2003 |
| | | US 6500979 B1 | 31-12-2002 |
| | | ZA 200002739 A | 11-12-2000 |
| EP 1106594 A | 13-06-2001 | DE 19957528 A1 | 31-05-2001 |
| | | BR 0005637 A | 17-07-2001 |
| | | CA 2327022 A1 | 30-05-2001 |
| | | CN 1297876 A | 06-06-2001 |
| | | EP 1106594 A2 | 13-06-2001 |
| | | JP 2001163820 A | 19-06-2001 |
| | | PL 344211 A1 | 04-06-2001 |
| | | SG 97975 A1 | 20-08-2003 |
| | | US 2001003785 A1 | 14-06-2001 |
| | | ZA 200007014 A | 05-06-2001 |
| EP 1106596 A | 13-06-2001 | DE 19957522 A1 | 31-05-2001 |
| | | BR 0005672 A | 27-11-2001 |
| | | CA 2327047 A1 | 30-05-2001 |
| | | CN 1297877 A | 06-06-2001 |
| | | EP 1106596 A2 | 13-06-2001 |
| | | JP 2001163823 A | 19-06-2001 |
| | | PL 344210 A1 | 04-06-2001 |
| | | SG 86452 A1 | 19-02-2002 |
| | | TW 548264 B | 21-08-2003 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 05 10 0167

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-07-2005

| Im Recherchenbericht angeführtes Patentdokument | Datum der Veröffentlichung | Mitglied(er) der Patentfamilie | Datum der Veröffentlichung |
|---|---|---|---|
| EP 1106596 A | | US 6340778 B1 | 22-01-2002 |
| | | ZA 200007013 A | 07-06-2001 |

EPO FORM P0461

Für nähere Einzelheiten zu diesem Anhang : siehe Amtsblatt des Europäischen Patentamts, Nr.12/82